# EUROPÄISCHE PATENTANMELDUNG

(11) **EP 1 300 132 A2**
(43) Veröffentlichungstag der Anmeldung: **09.04.2003**
(21) Anmeldenummer: 02021427.6
(22) Anmeldetag: 25.09.2002
(51) Int. Cl.: A61K 7/13, D06P 3/14

(54) **Mittel zum Färben von keratinhaltigen Fasern**

(30) Priorität: 04.10.2001 DE 10148849
(71) Anmelder: Henkel Kommanditgesellschaft auf Aktien, 40589 Düsseldorf-Holthausen (DE)
(72) Erfinder: Möller, Hinrich, Dr., 40789 Monheim (DE); Oberkobusch, Doris, Dr., 40591 Düsseldorf (DE); Höffkes, Horst, Dr., 40595 Düsseldorf (DE)

(57) **Zusammenfassung**

Es wird ein Mittel zum Färben von keratinhaltigen Fasern, insbesondere menschlichen Haaren, beansprucht, enthaltend
**A.** als Komponente A mindestens ein Dicyanmethylenindan-Derivat gemäß Formel I und/oder deren physiologisch verträgliches Salz, 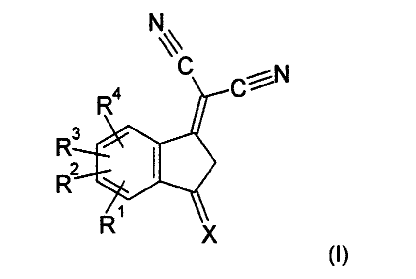 wobei
   - R¹, R², R³ und R⁴ stehen unabhängig voneinander für ein Wasserstoffatom, ein Halogenatom, eine C₁-C₄-Alkylgruppe, eine Aryl-C₁-C₄-alkylgruppe, eine C₁-C₄-Alkoxycarbonylgruppe, eine C₁-C₄-Acyloxygruppe, eine C₁-C₄-Hydroxyalkoxygruppe, eine gegebenenfalls substituierte Benzyloxygruppe, eine C₁-C₄-Carboxyalkylgruppe, eine Hydroxygruppe, eine Nitrogruppe, eine Carboxygruppe, eine Sulfonsäuregruppe, eine Sulfamoylgruppe, eine C₁-C₄-Acylaminogruppe, eine Gruppe R^{I}R^{II}N -, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe, wobei zwei der Gruppen R¹, R², R³ und R⁴ zusammen mit dem Benzolrest des Indan-Grundgerüstes einen ankondensierten, aromatischen Ring bilden können,
   - X steht für ein Sauerstoffatom oder eine Dicyanmethylengruppe,
**B.** und gegebenenfalls als Komponente B mindestens eine Verbindung ausgewählt aus einer Gruppe, die gebildet wird, aus
   (a) Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen,
   (b) Aminosäuren,
   (c) aus 2 bis 9 Aminosäuren aufgebauten Oligopeptiden,
   (d) CH-aciden Verbindungen,
   (e) aromatischen und heteroaromatischen Aldehyden bzw. Ketonen und
   (f) quartären Ammoniumverbindungen,
   mit der Maßgabe, daß, wenn X eine Dicyanmethylengruppe ist, zwingend eine Komponente B enthalten ist,
und sich auch in Abwesenheit von oxidierenden Agentien hervorragend zum Färben von keratinhaltigen Fasern eignet.

## Beschreibung

Die Erfindung betrifft ein Mittel zum Färben von keratinhaltigen Fasern, insbesondere menschlichen Haaren, das Dicyanmethylenindan-Derivate enthält, die Verwendung dieser Derivate in Mitteln zum Färben von keratinhaltigen Fasern sowie ein Verfahren zum Färben von keratinhaltigen Fasern, insbesondere menschlichen Haaren.

Für das Färben von keratinhaltigen Fasem kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxyoder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken.

Direktziehende Farbstoffe werden unter schonenderen Bedingungen appliziert. Oftmals werden sie zur Nuancierung von Oxidationsfärbungen verwendet, da die Oxidationsfarbstoffe die Farbpalette, insbesondere im Rot- und Blaubereich, noch nicht zufriedenstellend abdecken. Der Nachteil der Verwendung direktziehender Farbstoffe liegt jedoch darin, daß die Färbungen häufig nur über unzureichende Echtheitseigenschaften verfügen.

Färbemittel mit Dicyanmethylenindan-Derivaten und deren Verwendung zum Färben von keratinhaltigen Fasern sind bislang aus der EP-A-1 002 516 bekannt. Die dort offenbarten 1,3-Bis(Dicyanmethylen)-indan-Derivate färben als direktziehende Farbstoffe Keratinfasern blau. In Kombination mit weiteren direktziehenden Farbstoffen lassen sich graue, braune und blonde Färbungen erzielen.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Die Farbpalette soll, insbesondere um intensive Rottöne erweitert werden.

Es wurde nun gefunden, daß die in der Formel I dargestellten Dicyanmethylenindan-Derivate sich hervorragend zum Färben von keratinhaltigen Fasern eignen. Sie ergeben in Kombination mit Reagenzien wie beipielsweise Carbonylverbindungen Ausfärbungen mit hervorragender Brillanz und Farbtiefe, insbesondere im Rotbereich. Ausfärbungen über einen Nuancenbereich von gelb über gelbbraun, orange, braunorange, braun, rot, rotviolett, blau bis hin zu schwarz sind möglich.

Gegenstand der Erfindung ist ein Mittel zum Färben von keratinhaltigen Fasern, insbesondere menschlichen Haaren, enthaltend
**A.** als Komponente A mindestens ein Dicyanmethylenindan-Derivat gemäß Formel I und/oder deren physiologisch verträgliches Salz, 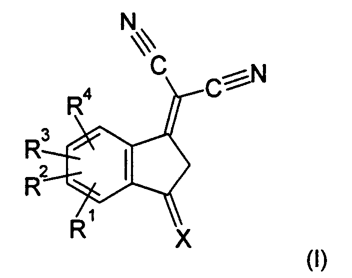 wobei
   - R¹, R², R³ und R⁴ stehen unabhängig voneinander für ein Wasserstoffatom, ein Halogenatom, eine C₁-C₄-Alkylgruppe, eine Aryl-C₁-C₄-alkylgruppe, eine C₁-C₄-Alkoxycarbonylgruppe, eine C₁-C₄-Acyloxygruppe, eine C₁-C₄-Hydroxyalkoxygruppe, eine gegebenenfalls substituierte Benzyloxygruppe, eine C₁-C₄-Carboxyalkylgruppe, eine Hydroxygruppe, eine Nitrogruppe, eine Carboxygruppe, eine Sulfonsäuregruppe, eine Sulfamoylgruppe, eine C₁-C₄-Acylaminogruppe, eine Gruppe R'R"N-, worin R' und R" stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe,
      wobei zwei der Gruppen R¹, R², R³ und R⁴ zusammen mit dem Benzolrest des Indan-Grundgerüstes einen ankondensierten, aromatischen Ring bilden können,
      - X steht für ein Sauerstoffatom oder eine Dicyanmethylengruppe,
**B.** und gegebenenfalls als Komponente B mindestens eine Verbindung ausgewählt aus einer Gruppe, die gebildet wird, aus
   (a) Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen,
   (b) Aminosäuren,
   (c) aus 2 bis 9 Aminosäuren aufgebauten Oligopeptiden,
   (d) CH-aciden Verbindungen,
   (e) aromatischen und heteroaromatischen Aldehyden bzw. Ketonen und
   (f) quartären Ammoniumverbindungen,
mit der Maßgabe, daß, wenn X eine Dicyanmethylengruppe ist, zwingend eine Komponente B enthalten ist.

Die Verbindungen mit der Formel I können auch in Form ihrer Tautomere oder als Carbanionen vorliegen. Liegen die Dicyanmethylenindan-Derivate als Carbanionen vor, dienen als Gegenionen bevorzugt Alkali- oder Erdalkalimetallkationen, sowie Ammoniumionen. Die Gegenionen der sonstigen physiologisch verträglichen Salze der Verbindungen der Formel I, wie z.B. der Carboxylate und der Sulfonate, sind ebenso vorzugsweise Alkali-, Erdalkali- und Ammoniumionen.

Beispiele für die als Substituenten im Rahmen dieser Anmeldung genannten C₁-C₄-Alkylreste sind die Gruppen Methyl, Ethyl, Propyl, Isopropyl, n-Butyl, sec-Butyl und tert.-Butyl. Ethyl und Methyl sind bevorzugte Alkylreste. Bevorzugte C₂-C₆-Alkenylgruppen sind die Vinyl-, die Allyl- und die Butenylgruppe. Erfindungsgemäß bevorzugte C₁-C₄-Alkoxyreste sind beispielsweise eine Methoxy-, eine Ethoxy- oder eine Butoxygruppe. Weiterhin können als bevorzugte Beispiele für eine C₁-C₄-Hydroxyalkylgruppe eine Hydroxymethyl-, eine 2-Hydroxyethyl-, eine 2-Hydroxypropyl, eine 3-Hydroxypropyl- und eine 4-Hydroxybutylgruppe genannt werden. Eine 2-Hydroxyethylgruppe ist besonders bevorzugt. Eine bevorzugte Hydroxy-C₁-C₄-Alkoxygruppe ist die 2-Hydroxyethoxygruppe. Die Acetylgruppe, Propionylgruppe und Butyrylgruppe sind bevorzugte C₁-C₄-Acylgruppen. Eine bevorzugte C₁-C₄-Carboxyalkylgruppe ist die 3-Carboxypropylgruppe. Bevorzugte C₁-C₄-Acyloxygruppen sind Acetyloxy und Propionyloxy. Bevorzugte C₁-C₄-Alkoxycarbonylgruppen sind Methoxycarbonyl, Ethoxycarbonyl, Propoxycarbonyl, Isopropoxycarbonyl und Butoxycarbonyl. Beispiele für eine C₁-C₄-Acylaminogruppe sind die Acetylamino- und die Propionylaminogruppe. Beispiele für Halogenatome sind Fluor-, Chlor-, Brom-, oder lodatome, wobei Chlor- und Bromatome besonders bevorzugt sind. Bevorzugte C₁-C₄-Aminoalkylgruppen sind die Aminomethyl-, die Aminoethyl und die Aminopropylgruppe. Beispiele für eine Aryl-C₁-C₄-alkylgruppe sind Benzyl und 2-Phenylethyl. Die Dimethylamino-, Diethylamino- und die Aminogruppe sind Beispiele für eine Gruppe R'R"N-. Die oben genannten Beispiele der verwendeten Reste sind auch für die folgenden Formeln II, III und IV relevant. Die weiteren verwendeten Begriffe leiten sich erfindungsgemäß von den hier gegebenen Definitionen ab.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyloder Acetylcellulose und synthetischer Fasern, wie z. B. Polyamid-, Polyacrylnitril-, Polyurethan- und Polyesterfasem verwendet werden.

In einer bevorzugten Ausführungsform steht X für ein Sauerstoffatom.

Weiterhin ist es bevorzugt, die Komponente B aus aromatischen und heteroaromatischen Aldehyden bzw. Ketonen auszuwählen, wenn X eine Dicyanmethylengruppe ist.

Vorzugsweise sind die Dicyanmethylenindan-Derivate mit der Formel I ausgewählt aus 3-Dicyanmethylen-1-indanon, 4-Acetylamino-3-dicyanmethylen-1-indanon, 5-Acetylamino-3-dicyanmethylen-1-indanon, 4-Brom-3-dicyanmethylen-1-indanon, 5-Brom-3-dicyanmethylen-1-indanon, 4-Chlor-3-dicyanmethylen-1-indanon, 5-Chlor-3-dicyanmethylen-1-indanon, 4,5-Dichlor-3-dicyanmethylen-1-indanon, 4,6-Dichlor-3-dicyanmethylen-1 -indanon,- 5-Carboxy-3-dicyanmethylen-1-indanon, 3-Dicyanmethylen-4-fluor-1-indanon, 3-Dicyanmethylen-5-fluor-1-indanon, 3-Dicyanmethylen-4-nitro-1-indanon, 3-dicyanmethylen-5-nitro-1-indanon, 3-Dicyanmethylen-5-hydroxy-1-indanon, 3-Dicyanmethylen-4-methoxy-1-indanon, 3-Dicyanmethylen-5-methoxy-1-indanon, 3-Dicyanmethylen-4,6-dimethoxy-1-indanon, 3-Dicyanmethylen-5-methyl-1-indanon, 3-Dicyanmethylen-4,5-dimethyl-1-indanon, 1,3-Bis(dicyanmethylen)-indan, 4-Brom-1,3-bis(dicyanmethylen)-indan, 5-Brom-1,3-bis(dicyanmethylen)-indan, 4,5,6,7-Tetrabrom-1,3-bis(dicyanmethylen)-indan, 4-Chlor-1,3-bis(dicyanmethylen)-indan, 5-Chlor-1,3-bis(dicyanmethylen)-indan, 4,5-Dichlor-1,3-bis(dicyanmethylen)-indan, 4,6-Dichlor-1,3-bis(dicyanmethylen)-indan, 4,5,6,7-Tetrachlor-1,3-bis(dicyanmethylen)-indan, 5-Carboxy-1,3-bis(dicyanmethylen)-indan, 5-Butoxy-1,3-bis(dicyanmethylen)-indan, 4-Benzyloxy-1,3-bis(dicyanmethylen)-indan, 5-Benzyloxy-1,3-bis(dicyanmethylen)-indan, 4-(4-Methoxybenzyloxy)-1,3-bis(dicyanmethylen)-indan, 4-Acetoxy-5,7-dichlor-1,3-bis(dicyanmethylen)-indan, 4-Amino-1,3-bis(dicyanmethylen)-indan, 5-Amino-1,3-bis(dicyanmethylen)-indan, 4-Acetylamino-1,3-bis(dicyanmethylen)-indan, 5-Acetylamino-1,3-bis(dicyanmethylen)-indan, 1,3-Bis(dicyanmethylen)-4-fluor-indan, 1,3-Bis(dicyanmethylen)-5-fluor-indan, 1,3-Bis (dicyanmethylen)-4-iod-indan, 1,3-Bis(dicyanmethylen)-4-nitro-indan, 1,3-Bis(dicyanmethylen)-5-nitro-indan, 1,3-Bis(dicyanmethylen)-5-hydroxy-indan, 1,3-Bis(dicyanmethylen)-5-methoxy-indan, 1,3-Bis(dicyanmethylen)-4-methoxy-indan, 1,3-Bis(dicyanmethylen)-4,6-dimethoxy-indan, 1,3-Bis(dicyanmethylen)-5,6-dimethoxy-indan, 1,3-Bis(dicyanmethylen)-4,5,6-trimethoxyindan, 1,3-Bis(dicyanmethylen)-4-ethoxy-indan, 1,3-Bis(dicyanmethylen)-5-ethoxy-indan, 1,3-Bis(dicyanmethylen)-5-methyl-indan, 1,3-Bis(dicyanmethylen)-4,5-dimethyl-indan, 1,3-Bis(dicyanmethylen)-5-ethyl-indan, 1,3-Bis(dicyanmethylen)-benzo[f]indan, sowie deren physiologisch verträglichen Salzen.

Dicyanmethylenindan-Derivate der Formel I sind zum großen Teil literaturbekannt, im Handel erhältlich oder nach bekannten Syntheseverfahren herstellbar.

In einer weiteren Ausführungsform enthält das Färbemittel mindestens ein Reaktionsprodukt aus einem Dicyanmethylenindan-Derivat mit der Formel I und einer Verbindung der Komponente B als direktziehenden Farbstoff. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Die voranstehend genannten Verbindungen mit der Formel I, die Verbindungen der Komponente B werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet. Sie können in Gegenwart von üblichen Oxidationsfarbstoffvorprodukten in Gegenwart eines Oxidationsmittels, wie beispielsweise Wasserstoffperoxid, eingesetzt werden. Die Reaktionsprodukte aus den beiden vorgenannten Verbindungen können als direktziehende Färbemittel in den gleichen Konzentrationen eingesetzt werden.

Geeignete Verbindungen mit primärer oder sekundärer Aminogruppe der Komponente B sind beispielsweise primäre und sekundäre aromatische Amine wie N,N-Dimethyl-pphenylendiamin, N,N-Diethyl-p-phenylendiamin, N-(2-Hydroxyethyl)-N-ethyl-pphenylendiamin, N,N-Bis-(2-hydroxyethyl)-p-phenylendiamin, N-(2-Methoxyethyl)-pphenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 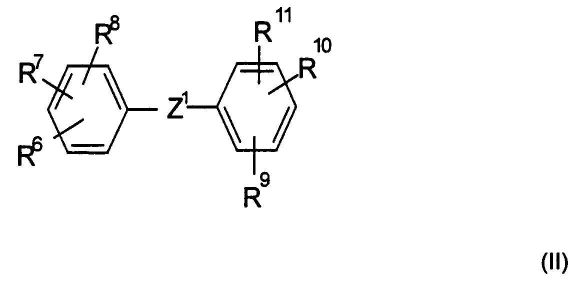 in der
- R⁶ steht für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann,
- R⁷, R⁸, R⁹, R¹⁰ und R¹¹ stehen unabhängig voneinander für ein Wasserstoffatom, eine Hydroxy- oder eine Aminogruppe, die durch C₁₋₄-Alkyl-, C₁₋₄-Hydroxyalkyl-, C₁₋₄-Alkoxy-, C₁₋₄-Aminoalkyl- oder C₁₋₄-Alkoxy-C₁₋₄-alkylgruppen substituiert sein kann,
- Z¹ steht für eine direkte Bindung, eine gesättigte oder ungesättigte, gegebenenfalls durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel III

   **-Z**^{**2**}**-(CH**_{**2**}**-Z**^{**4**}**-CH**_{**2**}**-Z**^{**3**}**)**_{**o**}**-** (III)
in der
- Z⁴ eine direkte Bindung, eine CH₂- oder CHOH-Gruppe bedeutet,
- Z² und Z³ unabhängig voneinander für ein Sauerstoffatom, eine NR¹⁴-Gruppe, worin R¹⁴ Wasserstoff, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p'2 oder 3 sind, stehen und
- o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5;6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprotein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Prolin, Lysin und Tryptophan. Aber auch andere Aminosäuren, wie z.B. 6-Aminocapronsäure und β-Alanin, können eingesetzt werden. Die physiologisch verträglichen Salze der vorgenannten Verbindungen sind ebenso umfaßt.

Als CH-acide Verbindungen der Komponente B können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazoliump-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-lndoxylacetat, 2-Cumaranon, 5-Hydroxy-cumaranon, 6-Hydroxy-cumaranon, 1-Methyl-3-phenyl-2-pyrazolinon, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolinhydrochlorid, 3-Cyan-1,4-dimethyl-6-hydroxy-2-pyridon und 3-Cyan-1-ethyl-6-hydroxy-4-methyl-2-pyridon.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z.B. Glutathion oder die in den Hydrolysaten von Kollagen, Keratin, Casein, Elastin, Sojaprotein, Weizengluten oder Mandelprotein enthaltenen Oligopeptide zu nennen. Bevorzugt ist dabei die Verwendung gemeinsam mit Verbindungen mit primärer oder sekundärer Aminogruppe oder mit aromatischen Hydroxyverbindungen.

Beispiele für quartäre Ammoniumsalze sind Tetramethyl-, Tetraethyl-, Tetrapropyl-, Tetrabutyl-, Benzyltrimethylammonium-chlorid, -bromid, -iodid, -hydrogensulfat, 0.5 - sulfat sowie den quaternierten Hydroxyethylcellulose-Derivaten (INCl-Bezeichnung: Polyquaternium 10).

Die erfindungsgemäß eingesetzten aromatischen und heteroaromatischen Aldehyde bzw. Ketone der Komponente B sind ausgewählt aus Verbindungen gemäß Formel IV, 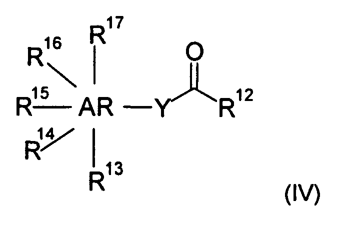 wobei
- AR steht für Benzol, Naphthalin, Pyridin, Pyrimidin, Pyrazin, Pyrazidin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, Cyclo-C₅-C₇-alkyl-on, wobei die N-Heteroaromaten auch quaterniert sein können,
- R¹² steht für ein Wasserstoffatom, eine C₁-C₄-Alkyl-, C₁-C₄-Acyl-, C₂-C₄-Alkenyl-, eine Aryl-C₂-C₄-alkenyl-, C₁-C₄-Perfluoralkyl-, eine ggf. substituierte Aryl- oder Heteroarylgruppe oder eine gegebenefalls substituierte Carbamoylgruppe,
- R¹³, R¹⁴, R¹⁵, R¹⁶ und R¹⁷ stehen unabhängig voneinander für ein Wasserstoffatom, ein Halogenatom, eine C₁-C₄-Alkyl-, C₁₋₄-Alkoxy-, C₁-C₄-Aminoalkyl-, C₁-C₄-Hydroxyalkylgruppe, eine C₁-C₄-Acylgruppe, eine Carboxy-, Carboxylato-, Sulfo-, Sulfato-, C₂-C₆-Alkenyl-, Aryl-, eine Aryl-C₁-C₄-alkylgruppe, eine Hydroxy-, Nitro-, Pyrrolidino, Morpholino-, Piperidino-, Amino- bzw. Ammonio- oder 1-lmidazoliniogruppe, wobei die letzten drei Gruppen mit C₁-C₄-Alkyl-, C₁-C₄-Carboxyalkyl-, C₁-C₄-Hydroxyalkyl-, C₁-C₄-Hydroxyalkoxygruppen, C₂-C₆-Alkenylgruppen, mit ggf. substituierten Benzylgruppen, mit Sulfo-(C₁-C₄)-alkyl-, Amino-C₁-C₄-alkyl-, Mono-C₁-C₄-alkylamino-C₁-C₄-alkyl-, Di-C₁-C₄-alkylamino-C₁-C₄-alkyl- oder Heterozyklus-(C₁-C₄)-alkylgruppen substituiert sein können, wobei auch zwei der Reste -Y-CO-R¹², R¹³, R¹⁴, R¹⁵, R¹⁶ und R¹⁷ zusammen einen ankondensierten gegebenenfalls substituierten 5-, 6- oder 7-Ring, der ebenfalls einen oder zwei ankondensierte aromatische Ringe tragen kann, bilden können, wobei das System AR in Abhängigkeit von der Größe des Ringes weitere Substituenten tragen kann, die unabhängig voneinander für die gleichen Gruppen stehen können wie R¹³, R¹⁴, R¹⁵, R¹⁶ und R¹⁷,
- Y steht für eine direkte Bindung, eine Carbonyl-, Methylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei X zusammen mit der CO-R¹-Gruppe auch einen gegebenenfalls substituierten 5-, 6- oder 7-Ring bilden kann.

Bevorzugte Verbindungen der Formel IV der Komponente B sind ausgewählt aus
- 5-(4-Dimethylaminophenyl)-penta-2,4-dienal, 5-(4-Diethylaminophenyl)-penta-2,4-dienal, 5-(4-Methoxyphenyl)-penta-2,4-dienal, 5-(3,4-Dimethoxyphenyl)-penta-2,4-dienal, 5-(2,4-Dimethoxyphenyl)-penta-2,4-dienal, 5-(4-Piperidinophenyl)-penta-2,4-dienal, 5-(4-Morpholinophenyl)-penta-2,4-dienal, 5-(4-Pyrrolidinophenyl)-penta-2,4-dienal, 6-(4-Dimethylaminophenyl)-hexa-2,4-dien-2-on, 6-(4-Diethylaminophenyl)-hexa-2,4-dien-2-on, 6-(4-Methoxyphenyl)-hexa-2,4-dien-2-on, 6-(3,4-Dimethoxyphenyl)-hexa-2,4-dien-2-on, 6-(2,4-Dimethoxyphenyl)-hexa-2,4-dien-2-on, 6-(4-Piperidinophenyl)-hexa-2,4-dien-2-on, 6-(4-Morpholinophenyl)-hexa-2,4-dien-2-on, 6-(4-Pyrrolidinophenyl)-hexa-2,4-dien-2-on, 5-(4-Dimethylaminonaphth-1-yl)-penta-2,4-dienal,
- 2-Nitropiperonal, 5-Nitropiperonal, 6-Nitropiperonal, 5-Hydroxy-2-nitropiperonal, 2-Hydroxy-5-nitropiperonal, 2-Chlor-6-nitropiperonal, 5-Chlor-2-nitropiperonal, 2,6-Dinitropiperonal,
- 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4-Acetylphenyl)-trimethylammonium-, 4-(N,N-Diethyl)-N-methylammonio)-benzaldehyd-, N-(4-Benzoylphenyl)-trimethylammonium-, N-(4-Benzoylphenyl)-N,N-diethylmethylammonium-, N-(4-Formylphenyl)-N-methylpyrrolidinium-, N-(4-Formylphenyl)-N-methylpiperidinium-, N-(4-Formylphenyl)-N-methylmorpholinium-, N-(4-Acetylphenyl)-N-methylmorpholinium-, N-(4-Benzoylphenyl)-N-methylmorpholinium-, 3-Formyl-N-ethyl-N-methylcarbazolium-, 3-Formyl-9,9-dimethylcarbazolium-, 1-(4-Acetylphenyl)-3-methylimidazolium-, 1-(4-Acetylphenyl)-3-methyl-2-imidazolinium-, 1-(4-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, lodide, Tetrachlorozinkate, Methylsulfate, Trifluormethansulfonate, Hexafluorophosphate, Tetrafluoroborate,
- 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1 -benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2-Formylvinyl)-1-methylpyridinium-, 1,3-Dimethyl-2-(4-formylphenyl)-benzimidazolinium-, 1,3-Dimethyl-2-(4-formylphenyl)-imidazolinium-, 2-(4-Formylphenyl)-3-methylbenzothiazolium-, 2-(4-Acetylphenyl)-3-methylbenzothiazolium-, 2-(4-Formylphenyl)-3-methylbenzoxazolium-, 2-(5-Formyl-2-furyl)-3-methylbenzothiazolium-, 2-(5-Formyl-2-thienyl)-3-methylbenzothiazolium-, 2-(3-Formylphenyl)-3-methylbenzothiazolium-, 2-(4-Formylnaphth-1-yl)-3-methylbenzothiazolium-, 5-Chlor-2-(4-formylphenyl)-3-methylbenzothiazolium-, 2-(4-Formylphenyl)-3,5-dimethylbenzothiazolium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium, 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-formylphenyl)-ethenyl]-chinolinium, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-, 1-Methyl-5-oxo-indeno[1,2-b]pyridinium-, 1-Ethyl-5-oxo-indeno[1,2-b]pyridinium-, 1-Benzyl-5-oxo-indeno[1,2-b]pyridinium-, 2-Methyl-5-oxo-indeno[1,2-c]pyridinium-, 2-Methyl-9-oxo-indeno[2,1-c]pyridinium-, 1 -Methyl-9-oxo-indeno[2,1-b]pyridiniumsalze, insbesondere Benzolsulfonat, p-Toluolsulfonat, Methansulfonat, Perchlorat, Sulfat, Chlorid, Bromid, lodid, Tetrachlorozinkat, Methylsulfat, Trifluormethansulfonat, Tetrafluoroborat,
- Salicylaldehyd, Vanillin, 4-Hydroxy-3-methoxyzimtaldehyd (Coniferylaldehyd), 2,4-Dihydroxybenzaldehyd, 4-Hydroxybenzaldehyd, 4-Dimethylaminobenzaldehyd, 4-Diethylaminobenzaldehyd, 4-Dimethylamino-2-hydroxybenzaldehyd, 4-Pyrrolidino-benzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)-benzaldehyd, 4-Diphenylaminobenzaldehyd, 2,3,6,7-Tetrahydro-1H,5Hbenzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-lmidazolyl)-benzaldehyd, 2-Morpholinobenzaldehyd,
- 1,3-Diacetylbenzol, 1,4-Diacetylbenzol, 1,3,5-Triacetylbenzol, 2-Benzoyl-acetophenon, 2-(4-Methoxybenzoyl)-acetophenon, 2-(2-Furoyl)-acetophenon, 2-(2-Pyridoyl)-acetophenon, 2-(3-Pyridoyl)-acetophenon,
- 1-Phenyl-1,2-propandion, 1-Phenyl-1,2-butandion, 1-(Chlorphenyl)-1,2-propandion, 1-Phenyl-3,3-dimethyl-1,2-butandion, Benzil, Anisil, Salicil, 5,5'-Dibromsalicil, 2,2'-Furil-, 2,2'-Thienil-, 2,2'-Pyridil, 4,4'-Pyridil, 6,6'-Dimethyl-4,4'-pyridil, 4-Hydroxy-benzil, 4-Methoxy-benzil, 4-Chlor-benzil, 4-Methyl-benzil, 4-Dimethylamino-benzil, 4,4'-Dihydroxy-benzil, 4,4'-Dimethyl-benzil, 4,4'-Dibrom-benzil, 4,4'-Dichlor-benzil, 4,4'-Bis-dimethylamino-benzil, 2,4-Dihydroxy-benzil, 3,3'-Dimethoxy-benzil, 2'-Chlor-3,4-dimethoxy-benzil, 3,4,5,3',4',5'-Hexamethoxybenzil,
- Isatinderivate, wie 5-Chlorisatin, 5-Methoxyisatin, 5-Nitroisatin, 6-Nitroisatin, Isatin-5-sulfonsäure, Isatin-4-carbonsäure und Isatin-5-carbonsäure,
- N-substituierte Isatin-Derivate, wie N-Methylisatin, N-(2-Hydroxyalkyl)-isatin, N-(2-Hydroxypropyl)-isatin, N-(3-Hydroxypropyl)-isatin, N-(2,3-Dihydroxypropyl)-isatin, N-(2-Sulfoethyl)-isatin, (3-Sulfopropyl)-isatin, N-Allylisatin, N-Vinylisatin, N-Benzylisatin, N-(4-Methoxybenzyl)-isatin, N-(4-Carboxybenzyl)-isatin, N-(4-Sulfobenzyl)-isatin, N-(2-Dimethylaminoethyl)-isatin, N-(2-Pyrrolidinoethyl)-isatin, N-(2-Piperidinoethyl)-isatin, (2-Morpholinoethyl)-isatin, N-(2-Furylmethyl)-isatin, N-(Thien-2-ylmethyl)-isatin; N-(Pyrid-2-ylmethyl)-isatin, N-(Pyrid-3-ylmethyl)-isatin, N-(Pyrid-4-ylmethyl)-isatin, N-Allylisatin-5-sulfonsäure, 5-Chlor-N-(2-hydroxyethyl)-isatin, 5-Methyl-N-(2-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2-hydroxyethyl)-aminomethyl)-isatin, N-(2-Hydroxyethylaminomethyl)-isatin, N-(Bis-(2-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2-Carboxyethylaminomethyl)-isatin, N-(3-Carboxypropylaminomethyl)-isatin, N-(Bis-(2-hydroxyethyl)-aminomethyl)-5-methylisatin, N-Piperidinomethyl-5-chlorisatin, N-(2-Sulfoethylamino)-isatin, sowie die Alkali- und gegebenenfalls Ammoniumsalze der sauren Verbindungen,
   Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxy-acetophenon, 4-Amino-acetophenon, 4-Dimethylamino-acetophenon, 4-Morpholino-acetophenon, 4-Piperidino-acetophenon, 4-lmidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, Indanon, 9-Fluorenon, 3-Hydroxyfluorenon, Anthron, 1,8-Dihydroxyanthron,
- heterocyclische Carbonylverbindungen, wie 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd, Thiophen-2-aldehyd, 5-Methylthiophen-2-aldehyd, 5-Nitrothiophen-2-aldehyd, Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
sowie den physiologisch verträglichen Salzen der voranstehenden Verbindungen.

Die Verbindungen der Komponente B werden besonders bevorzugt ausgewählt aus der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-ptoluolsulfonat, Thiobarbitursäure, Rhodanin, 2,3-Dimethyl-benzothiazolium-ptoluolsulfonat, 1,2-Dimethyl-naphtho[1,2-d]thiazolium-p-toluolsulfonat, 1-Methyl-2-chinaldinium-p-toluolsulfonat, 1,4-Dimethylchinolinium-p-toluolsulfonat, β-Alanin, L-Prolin, L-Lysin, DL-Tyrosin sowie deren vorzugsweise mit anorganischen Säuren gebildeten physiologisch verträglichen Salze, Vanillin, 5-Nitrovanillin, 4-Hydroxybenzaldehyd, 2,4-Dihydroxybenzaldehyd, 2,4-Dinitrobenzaldehyd, 4-Dimethylaminobenzaldehyd, 4-Pyrrolidino-benzaldehyd, 4-Diethylamino-2-hydroxybenzaldehyd, 4-Trimethylammoniobenzaldehyd-methansulfonat, 4-Trimethylammoniobenzaldehyd-p-toluolsulfonat, 4-Trimethylammoniobenzaldehyd-methylsulfat, 4-Hydroxynaphthaldehyd, 4-Dimethylaminonaphthaldehyd, Salicylaldehyd, 4-Dimethylaminozimtaldehyd, Piperonal, 5-Nitropiperonal, 4-Nitrosalicylaldehyd, Coniferylaldehyd, 4-Formyl-1-methylpyridinium-benzolsulfonat, 4-Formyl-1-methylpyridinium-p-toluolsulfonat, 4-Formyl-1-methylpyridinium-iodid, 4-Benzoyl-1-methylpyridinium-methansulfonat, 4-Benzoyl-1-methylpyridinium-p-toluolsulfonat, 4-Formyl-1-methyl-chinolinium-methansulfonat, 4-Formyl-1-methyl-chinolinium-ptoluolsulfonat, 4-Formyl-1-methyl-chinolinium-trifluormethansulfonat, 2-Formyl-1-methylchinolinium-methansulfonat, 2-Formyl-1-methyl-chinolinium-p-toluolsulfonat, 2-Formyl-1-methyl-chinolinium-trifluormethansulfonat, Chinolin-2-aldehyd, Chinolin-4-aldehyd, Indol-9-aldehyd, 1-Methyl-indol-3-aldehyd, 3-Azafluorenon, 3-Methyl-5-oxo-indano[2,1-b]pyridinium-p-toluolsulfonat, 3-Methyl-5-oxo-indano[2,1-b]pyridinium-trifluormethansulfonat, Antipyrin-4-aldehyd, Isatin, N-Vinylisatin, N-Allylisatin, Isatin-5-sulfonsäure (Natrium Salz), 5-(4-Dimethylaminophenyl)-penta-2,4-dienal, 9-Ethylcarbazol-3-carbaldehyd, 1,3-Diacetylbenzol, 1,3,5-Triacetylbenzol, 2-(2-Pyridoyl)-acetophenon, Salicil, 2,2'-Pyridil, 4-Hydroxyacetophenon, 2,4-Dihydroxyacetophenon, Furfural, 5-Nitrofurfural, 3-(5-Nitro-2-furyl)acrolein, Thiophen-2-aldehyd, 5-Nitrothiophen-2-aldehyd, 4-Pyridinaldehyd, 1-Methyl-4-[2-(4-formylphenyl)ethenyl]pyridinium-p-toluolsulfonat, 1-Methyl-4-[2-(4-formylphenyl)ethenyl]pyridinium-methylsulfat, 1-Methyl-2-[2-(4-formylphenyl)ethenyl]pyridinium-p-toluolsulfonat, 1-Methyl-2-[2-(4-formylphenyl)ethenyl]-pyridinium-methylsulfat, 2-(4-Formylphenyl)-3-methylbenzothiazolium-p-toluolsulfonat, 2-(4-Formylphenyl)-3-methylbenzothiazolium-methylsulfat, Pyridoxal und 2-(1,3,3,-Trimethyl-2-indolinyliden)acetaldehyd sowie den physiologisch verträglichen Salzen der vorgenannten Verbindungen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC_ Blue 2, Disperse Blue 3, Basic Blue 99, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis-(2-hydroxyethylamino)-2-nitrobenzolhydrochlorid und 1-Methyl-3-nitro-4-(2-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Ferner ist in einer weiteren Ausführungsform zusätzlich mindestens ein Oxidationsfarbstoffvorprodukt enthalten. Beispiele für solche Oxidationsfarbstoffvorprodukte wurden in der Einleitung bereits genannt.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-lmidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein zweiter Gegenstand der vorliegenden Erfindung betrifft die Verwendung von einer Kombination aus
**A.** Dicyanmethylenindan-Derivaten gemäß Formel I und/oder deren physiologisch verträglichen Salzen, 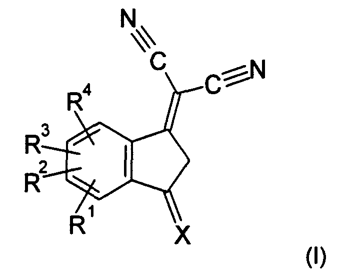 wobei R¹, R², R³, R⁴ und X wie oben definiert sind, und
**B.** einer Komponente B, die wie oben definiert ist,
als färbende Komponente in Haarfärbemitteln.

Wenn X für ein Sauerstoffatom steht, kann das Dicyanmethylenindan-Derivat der Formel I auch allein ohne die Komponente B als färbende Komponente in Haarfärbemitteln eingesetzt werden.

Darüber hinaus kann es bevorzugt sein, mindestens ein Reaktionsprodukt aus einem Dicyanmethylenindan-Derivat gemäß Formel I und einer Verbindung der Komponente B als färbende Komponenten in Haarfärbemitteln zu verwenden.

Ein dritter Gegenstand der vorliegenden Erfindung betrifft ein Verfahren zum Färben von keratinhaltigen Fasern, insbesondere menschlichen Haaren, worin ein Färbemittel, enthaltend
**A.** mindestens ein Dicyanmethylenindan-Derivat gemäß Formel I und/oder deren physiologisch verträglichen Salzen, 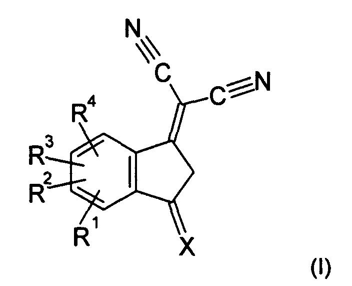 wobei R¹, R², R³, R⁴ und X wie oben definiert sind, und
**B.** eine Komponente B, die wie oben definiert ist,
   sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Dabei können die Dicyanmethylenindan-Derivate gemäß Formel I und die Verbindungen der Komponente B entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Wenn X für ein Sauerstoffatom steht, kann das Dicyanmethylenindan-Derivat der Formel I auch allein ohne die Komponente B in dem Verfahren zur Anwendung kommen.

Die Dicyanmethylenindan-Derivate gemäß Formel I und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

### Beispiele

### Herstellung einer Färbelösung

Es wurde je eine Aufschlämmung bzw. Lösung von 5 mmol des Dicyanmethylenindan-Derivats der Formel I (Komponente A) sowie 5 mmol der Verbindungen der Komponente B mit 5 mmol Natriumacetat in 50 ml Wasser bei ca. 50°C hergestellt. Die Aufschlämmungen bzw. Lösungen wurden nach Abkühlen auf 30°C miteinander vermischt und der pH-Wert auf pH 9 mit einer verdünnten wäßrigen NaOH-Lösung eingestellt.

### Ausfärbungen

In die frisch hergestellte Färbelösung wurde bei 30°C 30 Minuten lang eine Strähne zu 90% ergrauten, nicht vorbehandelten Menschenhaares eingebracht. Die Strähne wurde dann 30 Sekunden mit lauwarmem Wasser gespült, mit warmer Luft (30°C bis 40°C) getrocknet und anschließend ausgekämmt.

Die jeweiligen Farbnuancen und Farbtiefen der Beispielausfärbungen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

### Verbindungen der Komponente A (Tabelle 1):

- A1: 1,3-Bis(dicyanmethylen)-indan
- A2: 3-Dicyanmethylen-indan-1-on

### Verbindungen der Komponente B (Tabelle 1):

- B1: 4-Dimethylaminobenzaldehyd
- B2: 4-Hydroxybenzaldehyd
- B3: Vanillin
- B4: Coniferylaldehyd
- B5: 4-Formyl-1-methylpyridinium-benzolsulfonat
- B6: 4-Formyl-1-methylchinolinium-p-toluolsulfonat
- B7: 2-Formyl-1-methylchinolinium-p-toluolsulfonat
- B8: 2,4-Dinitrobenzaldehyd
- B9: 2,5-Diaminotoluol·H₂SO₄
- B10: 2,4,5,6-Tetraaminopyrimidin·2 H₂SO₄
- B11: 1,8-Bis-(2,5-diaminophenoxy)-3,6-dioxaoctan·4 HCI
- B12: 3-Amino-2-methylamino-6-methoxypyridin
- B13: 2-(2,5-Diaminophenoxy)-ethanol·H₂SO₄
- B14: 2-Aminomethyl-4-aminophenol-2 HCI
- B15: N,N-Bis(2-hydroxyethyl)-p-phenylendiamin·H₂SO₄
- B16: 4,4-Diaminodiphenylamin
- B17: 3,5-Diamino-2,6-dimethoxypyridin· 2 HCI
- B18: 4-Dimethylaminozimtaldehyd

**Tabelle 1**

| **Komponente A** | **Komponente B** | **Farbverstärker** | **Farbe** | **Farbtiefe** |
|---|---|---|---|---|
| A1 | - | - | blau | ++(+) |
| A1 | B1 | Piperidin | blau | ++(+) |
| A1 | B2 | Piperidin | blau | ++(+) |
| A1 | B3 | Piperidin | dunkelblau | +++ |
| A1 | B4 | Piperidin | dunkelbraun | +++ |
| A1 | B5 | Piperidin | hellblau | + |
| A1 | B6 | Piperidin | rotviolett | +(+) |
| A1 | B7 | Piperidin | blau | +(+) |
| A1 | B8 | Piperidin | dunkelblau | ++(+) |
| A2 | - | Piperidin | dunkelbraunviolett | ++(+) |
| A2 | B1 | Piperidin | dunkelrot | ++(+) |
| A2 | B2 | Piperidin | dunkelrot | +++ |
| A2 | B4 | Piperidin | dunkelviolettrot | +++ |
| A2 | B5 | Piperidin | schwarzrot | ++(+) |
| A2 | B6 | Piperidin | dunkelrot | ++(+) |
| A2 | B7 | Piperidin | schwarzrot | ++(+) |
| A2 | B8 | Piperidin | dunkelviolettrot | +++ |
| A2 | B9 | Piperidin | dunkelbraunviolett | +++ |
| A2 | B10 | Piperidin | dunkelbraunviolett | ++(+) |
| A2 | B11 | Piperidin | dunkelbraunviolett | ++(+) |
| A2 | B12 | Piperidin | dunkelviolettrot | +++ |
| A2 | B13 | Piperidin | dunkelviolettrot | +++ |
| A2 | B14 | Piperidin | dunkelviolettrot | +++ |
| A2 | B15 | Piperidin | dunkelviolettrot | +++ |
| A2 | B16 | Piperidin | dunkelrot | ++(+) |
| A2 | B17 | Piperidin | dunkelviolettrot | +++ |
| A2 | B18 | Piperidin | dunkelrot | ++(+) |

## Patentansprüche

1. Mittel zum Färben von keratinhaltigen Fasern, insbesondere menschlichen Haaren, enthaltend
**A.** als Komponente A mindestens ein Dicyanmethylenindan-Derivat gemäß Formel I und/oder deren physiologisch verträgliches Salz, 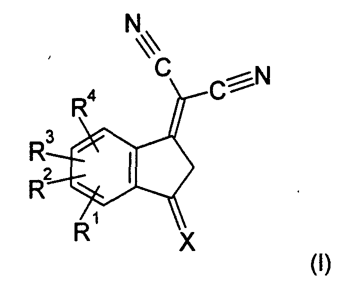 wobei
• R¹, R², R³ und R⁴ stehen unabhängig voneinander für ein Wasserstoffatom, ein Halogenatom, eine C₁-C₄-Alkylgruppe, eine Aryl-C₁-C₄-alkylgruppe, eine C₁-C₄-Alkoxycarbonylgruppe, eine C₁-C₄-Acyloxygruppe, eine C₁-C₄-Hydroxyalkoxygruppe, eine gegebenenfalls substituierte Benzyloxygruppe, eine C₁-C₄-Carboxyalkylgruppe, eine Hydroxygruppe, eine Nitrogruppe, eine Carboxygruppe, eine Sulfonsäuregruppe, eine Sulfamoylgruppe, eine C₁-C₄-Acylaminogruppe, eine Gruppe R'R"N-, worin R' und R" stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe,
wobei zwei der Gruppen R¹, R², R³ und R⁴ zusammen mit dem Benzolrest des Indan-Grundgerüstes einen ankondensierten aromatischen Ring bilden können,
• X steht für ein Sauerstoffatom oder eine Dicyanmethylengruppe,
**B.** und gegebenenfalls als Komponente B mindestens eine Verbindung ausgewählt aus einer Gruppe, die gebildet wird, aus
(a) Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen,
(b) Aminosäuren,
(c) aus 2 bis 9 Aminosäuren aufgebauten Oligopeptiden,
(d) CH-aciden Verbindungen,
(e) aromatischen und heteroaromatischen Aldehyden bzw. Ketonen und
(f) quartären Ammoniumverbindungen,
mit der Maßgabe, daß, wenn X eine Dicyanmethylengruppe ist, zwingend eine Komponente B enthalten ist.

2. Mittel nach Anspruch 1, **dadurch gekennzeichnet, daß** die Komponente B ausgewählt ist aus aromatischen und heteroaromatischen Aldehyden bzw. Ketonen, wenn X eine Dicyanmethylengruppe ist.

3. Mittel nach einem der Ansprüche 1 oder 2 , **dadurch gekennzeichnet, daß** die Dicyanmethylenindan-Derivate der Formel I ausgewählt sind aus 3-Dicyanmethylen-1-indanon, 4-Acetylamino-3-dicyanmethylen-1-indanon, 5-Acetylamino-3-dicyanmethylen-1-indanon, 4-Brom-3-dicyanmethylen-1-indanon, 5-Brom-3-dicyanmethylen-1-indanon, 4-Chlor-3-dicyanmethylen-1-indanon, 5-Chlor-3-dicyanmethylen-1-indanon, 4,5-Dichlor-3-dicyanmethylen-1-indanon, 4,6-Dichlor-3-dicyanmethylen-1-indanon, 5-Carboxy-3-dicyanmethylen-1-indanon, 3-Dicyanmethylen-4-fluor-1-indanon, 3-Dicyanmethylen-5-fluor-1-indanon, 3-Dicyanmethylen-4-nitro-1-indanon, 3-Dicyanmethylen-5-nitro-1-indanon, 3-Dicyanmethylen-5-hydroxy-1-indanon, 3-Dicyanmethylen-4-methoxy-1-indanon, 3-Dicyanmethylen-5-methoxy-1-indanon, 3-Dicyanmethylen-4,6-dimethoxy-1-indanon, 3-Dicyanmethylen-5-methyl-1-indanon, 3-Dicyanmethylen-4,5-dimethyl-1-indanon, 1,3-Bis(dicyanmethylen)-indan, 4-Brom-1,3-bis(dicyanmethylen)-indan, 5-Brom-1,3-bis(dicyanmethylen)-indan, 4,5,6,7-Tetrabrom-1,3-bis(dicyanmethylen)-indan, 4-Chlor-1,3-bis(dicyanmethylen)-indan, 5-Chlor-1,3-bis(dicyanmethylen)-indan, 4,5-Dichlor-1,3-bis(dicyanmethylen)-indan, 4,6-Dichlor-1,3-bis(dicyanmethylen)-indan, 4,5,6,7-Tetrachlor-1,3-bis(dicyanmethylen)-indan, 5-Carboxy-1,3-bis(dicyanmethylen)-indan, 5-Butoxy-1,3-bis(dicyanmethylen)-indan, 4-Benzyloxy-1,3-bis(dicyanmethylen)-indan, 5-Benzyloxy-1,3-bis(dicyanmethylen)-indan, 4-(4-Methoxybenzyloxy)-1,3-bis(dicyanmethylen)-indan, 4-Acetoxy-5,7-dichlor-1,3-bis(dicyanmethylen)-indan, 4-Amino-1,3-bis(dicyanmethylen)-indan, 5-Amino-1,3-bis(dicyanmethylen)-indan, 4-Acetylamino-1,3-bis(dicyanmethylen)-indan, 5-Acetylamino-1,3-bis(dicyanmethylen)-indan, 1,3-Bis(dicyanmethylen)-4-fluor-indan, 1,3-Bis(dicyanmethylen)-5-fluor-indan, 1,3-Bis(dicyanmethylen)-4-iod-indan, 1,3-Bis(dicyanmethylen)-4-nitro-indan, 1,3-Bis(dicyanmethylen)-5-nitro-indan, 1,3-Bis(dicyanmethylen)-5-hydroxy-indan, 1,3-Bis(dicyanmethylen)-5-methoxy-indan, 1,3-Bis(dicyanmethylen)-4-methoxy-indan, 1,3-Bis(dicyanmethylen)-4,6-dimethoxyindan, 1,3-Bis(dicyanmethylen)-5,6-dimethoxy-indan, 1,3-Bis(dicyanmethylen)-4,5,6-trimethoxy-indan, 1,3-Bis(dicyanmethylen)-4-ethoxy-indan, 1,3-Bis(dicyanmethylen)-5-ethoxy-indan, 1,3-Bis(dicyanmethylen)-5-methyl-indan, 1,3-Bis(dicyanmethylen)-4,5-dimethyl-indan, 1,3-Bis(dicyanmethylen)-5-ethyl-indan, 1,3-Bis(dicyanmethylen)-benzo[f]indan, sowie deren physiologisch verträglichen Salzen.

4. Mittel nach einem der Ansprüche 1 bis 3, **dadurch gekennzeichnet, daß** die Verbindungen der Formel I und die Verbindungen der Komponente B jeweils in einer Menge von 0,03 bis 65 mmol, insbesondere von 1 bis 40 mmol, bezogen auf 100 g des gesamten Färbemittels, enthalten sind.

5. Mittel nach einem der Ansprüche 1 bis 4, **dadurch gekennzeichnet, daß** die Komponente B ausgewählt ist aus primären und sekundären aromatischen Aminen wie N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr.7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan oder deren physiologisch verträglichen Salzen.

6. Mittel nach einem der Ansprüche 1 bis 5, **dadurch gekennzeichnet, daß** die Komponente B ausgewählt ist aus stickstoffhaltigen heterocyclischen Verbindungen ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxypyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin und Hydroxypyrimidin-Derivate, sowie die physiologisch vertäglichen Salze der vorgenannten Verbindungen.

7. Mittel nach einem der Ansprüche 1 bis 6, **dadurch gekennzeichnet, daß** Komponente B ausgewählt ist aus aromatischen Hydroxyverbindungen wie 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure sowie deren physiologisch verträglichen Salze.

8. Mittel nach einem der Ansprüche 1 bis 7, **dadurch gekennzeichnet, daß** die Komponente B ausgewählt ist aus Aminosäuren der Gruppe Arginin, Histidin, Tyrosin, Phenylalanin, DOPA (Dihydroxyphenylalanin), Ornithin, Prolin, Lysin, Tryptophan, 6-Aminocapronsäure und β-Alanin sowie deren physiologisch verträglichen Salze.

9. Mittel nach einem der Ansprüche 1 bis 8, **dadurch gekennzeichnet, daß** die Komponente B ausgewählt ist aus Oligopeptiden der Gruppe, die gebildet wird aus Glutathion und den in den Hydrolysaten von Kollagen, Keratin, Casein, Elastin, Sojaprotein, Weizengluten oder Mandelprotein enthaltenen Oligopeptiden.

10. Mittel nach einem der Ansprüche 1 bis 9, **dadurch gekennzeichnet, daß** die Komponente B ausgewählt ist aus CH-aciden Verbindungen der Gruppe 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolin-hydrochlorid, 3-Cyan-1,4-dimethyl-6-hydroxy-2-pyridon und 3-Cyan-1-ethyl-6-hydroxy-4-methyl-2-pyridon.

11. Mittel nach einem der Ansprüche 1 bis 10, **dadurch gekennzeichnet, daß** die Komponente B ausgewählt ist aus quartären Ammoniumsalzen der Gruppe Tetramethyl-, Tetraethyl-, Tetrapropyl-, Tetrabutyl-, Benzyltrimethylammonium-chlorid, -bromid, -iodid, -hydrogensulfat, 0.5 -sulfat sowie den quaternierten Hydroxyethylcellulose-Derivaten (INCl-Bezeichnung: Polyquaternium 10).

12. Mittel nach einem der Ansprüche 1 bis 11, **dadurch gekennzeichnet, daß** die Komponente B ausgewählt ist aus aromatischen oder heteroaromatischen Aldehyden bzw. Ketonen der Gruppe
- 5-(4-Dimethylaminophenyl)-penta-2,4-dienal, 5-(4-Diethylaminophenyl)-penta-2,4-dienal, 5-(4-Methoxyphenyl)-penta-2,4-dienal, 5-(3,4-Dimethoxyphenyl)-penta-2,4-dienal, 5-(2,4-Dimethoxyphenyl)-penta-2,4-dienal, 5-(4-Piperidinophenyl)-penta-2,4-dienal, 5-(4-Morpholinophenyl)-penta-2,4-dienal, 5-(4-Pyrrolidinophenyl)-penta-2,4-dienal, 6-(4-Dimethylaminophenyl)-hexa-2,4-dien-2-on, 6-(4-Diethylaminophenyl)-hexa-2,4-dien-2-on, 6-(4-Methoxyphenyl)-hexa-2,4-dien-2-on, 6-(3,4-Dimethoxyphenyl)-hexa-2,4-dien-2-on, 6-(2,4-Dimethoxyphenyl)-hexa-2,4-dien-2-on, 6-(4-Piperidinophenyl)-hexa-2,4-dien-2-on, 6-(4-Morpholinophenyl)-hexa-2,4-dien-2-on, 6-(4-Pyrrolidinophenyl)-hexa-2,4-dien-2-on, 5-(4-Dimethylaminonaphth-1-yl)-penta-2,4-dienal,
- 2-Nitropiperonal, 5-Nitropiperonal, 6-Nitropiperonal, 5-Hydroxy-2-nitropiperonal, 2-Hydroxy-5-nitropiperonal, 2-Chlor-6-nitropiperonal, 5-Chlor-2-nitropiperonal, 2,6-Dinitropiperonal,
- 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 5-Nitrovanillin, 3,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd,
- 4-Trimethylammoniobenzaldehyd-, 4-Benzyldimethylammoniobenzaldehyd-, 4-Trimethylammoniozimtaldehyd-, 4-Trimethylammonionaphthaldehyd-, 2-Methoxy-4-trimethylammoniobenzaldehyd-, N-(4-Acetylphenyl)-trimethylammonium-, 4-(N,N-Diethyl)-N-methylammonio)-benzaldehyd-, N-(4-Benzoylphenyl)-trimethylammonium-, N-(4-Benzoylphenyl)-N,N-diethylmethylammonium-, N-(4-Formylphenyl)-N-methylpyrrolidinium-, N-(4-Formylphenyl)-N-methylpiperidinium-, N-(4-Formylphenyl)-N-methylmorpholinium-, N-(4-Acetylphenyl)-N-methylmorpholinium-, N-(4-Benzoylphenyl)-N-methylmorpholinium-, 3-Formyl-N-ethyl-N-methylcarbazolium-, 3-Formyl-9,9-dimethylcarbazolium-, 1 -(4-Acetylphenyl)-3-methylimidazolium-, 1-(4-Acetylphenyl)-3-methyl-2-imidazolinium-, 1-(4-Benzoylphenyl)-3-methylimidazolium-, 5-Acetyl-1,3-diethyl-2-methylbenzimidazolium-, 5-Trimethylammonio-1-indanon-Salze, insbesondere die Benzolsulfonate, p-Toluolfulfonate, Methansulfonate, Ethansulfonate, Propansulfonate, Perchlorate, Sulfate, Chloride, Bromide, Iodide, Tetrachlorozinkate, Methylsulfate, Trifluormethansulfonate, Hexafluorophosphate, Tetrafluoroborate,
- 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2-Formylvinyl)-1-methylpyridinium-, 1,3-Dimethyl-2-(4-formylphenyl)-benzimidazolinium-, 1,3-Dimethyl-2-(4-formylphenyl)-imidazolinium-, 2-(4-Formylphenyl)-3-methylbenzothiazolium-, 2-(4-Acetylphenyl)-3-methylbenzothiazolium-, 2-(4-Formylphenyl)-3-methylbenzoxazolium-, 2-(5-Formyl-2-furyl)-3-methylbenzothiazolium-, 2-(5-Formyl-2-thienyl)-3-methylbenzothiazolium-, 2-(3-Formylphenyl)-3-methylbenzothiazolium-, 2-(4-Formylnaphth-1-yl)-3-methylbenzothiazolium-, 5-Chlor-2-(4-formylphenyl)-3-methylbenzothiazolium-, 2-(4-Formylphenyl)-3,5-dimethylbenzothiazolium-, 1-Methyl-2-[2-(4-formylphenyl)-ethenyl]-pyridinium-, 1-Methyl-4-[2-(4-acetylphenyl)-ethenyl]-pyridinium-, 1-Benzyl-4-[2-(4-formylphenyl)-ethenyl]-pyridinium, 1-Methyl-4-[2-(4-formylphenyl)-ethenyl]-pyridinium-, 1-Methyl-2-[2-(4-formylphenyl)-ethenyl]-pyridinium-, 1-Methyl-4-[2-(4-formylphenyl)-ethenyl]-chinolinium, 1-Methyl-2-[2-(4-formylphenyl)-ethenyl]-chinolinium-, 1-Methyl-2-[2-(5-formyl-2-furyl)-ethenyl]-chinolinium-, 1-Methyl-2-[2-(5-formyl-2-thienyl)-ethenyl]-chinolinium-, 1-Methyl-2-[2-(4-formylphenyl)-ethenyl]-benzothiazolinium-, 1,3-Dimethyl-2-[2-(4-formylphenyl)-ethenyl]-benzimidazolinium-, 1,3-Dimethyl-2-[2-(4-formylphenyl)-ethenyl]-imidazolinium-, 1-Methyl-5-oxo-indeno[1,2-b]pyridinium-, 1-Ethyl-5-oxoindeno[1,2-b]pyridinium-, 1-Benzyl-5-oxo-indeno[1,2-b]pyridinium-, 2-Methyl-5-oxo-indeno[1,2-c]pyridinium-, 2-Methyl-9-oxo-indeno[2,1-c]pyridinium-, 1-Methyl-9-oxo-indeno[2,1-b]pyridinium-salze, insbesondere Benzolsulfonat, p-Toluolsulfonat, Methansulfonat, Perchlorat, Sulfat, Chlorid, Bromid, lodid, Tetrachlorozinkat, Methylsulfat, Trifluormethansulfonat, Tetrafluoroborat,
- Salicylaldehyd, Vanillin, 4-Hydroxy-3-methoxyzimtaldehyd (Coniferylaldehyd), 2,4-Dihydroxybenzaldehyd, 4-Hydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 4-Piperidinobenzaldehyd, 4-Dimethylaminoacetophenon, 4-Hydroxynaphthaldehyd, 4-Dimethylaminonaphthaldehyd, 4-Dimethylaminobenzylidenaceton, 4-Dimethylaminozimtaldehyd, 2-Dimethylaminobenzaldehyd, 2-Chlor-4-dimethylaminobenzaldehyd, 4-Dimethylamino-2-methylbenzaldehyd, trans-4-Diethylamino-zimtaldehyd, 4-(Dibutylamino)-benzaldehyd, 4-Diphenylaminobenzaldehyd, 2,3,6,7-Tetrahydro-1H,5H-benzo[ij]chinolizin-9-carboxaldehyd, 4-Dimethylamino-2-methoxybenzaldehyd, 2,3,6,7-Tetrahydro-8-hydroxy-1H,5H-benzo[ij]chinolizin-9-carboxaldehyd, 4-(1-Imidazolyl)-benzaldehyd, 2-Morpholinobenzaldehyd,
- 1,3-Diacetylbenzol, 1,4-Diacetylbenzol, 1,3,5-Triacetylbenzol, 2-Benzoyl-acetophenon, 2-(4-Methoxybenzoyl)-acetophenon, 2-(2-Furoyl)-acetophenon, 2-(2-Pyridoyl)-acetophenon, 2-(3-Pyridoyl)-acetophenon,
- 1-Phenyl-1,2-propandion, 1-Phenyl-1,2-butandion, 1-(Chlorphenyl)-1,2-propandion, 1-Phenyl-3,3-dimethyl-1,2-butandion, Benzil, Anisil, Salicil, 5,5'-Dibromsalicil, 2,2'-Furil-, 2,2'-Thienil-, 2,2'-Pyridil, 4,4'-Pyridil, 6,6'-Dimethyl-4,4'pyridil, 4-Hydroxy-benzil, 4-Methoxy-benzil, 4-Chlor-benzil, 4-Methyl-benzil, 4-Dimethylamino-benzil, 4,4'-Dihydroxy-benzil, 4,4'-Dimethyl-benzil, 4,4'-Dibrom-benzil, 4,4'-Dichlor-benzil, 4,4'-Bis-dimethylamino-benzil, 2,4-Dihydroxy-benzil, 3,3'-Dimethoxy-benzil, 2'-Chlor-3,4-dimethoxy-benzil, 3,4,5,3',4',5'-Hexamethoxybenzil,
- Isatinderivate, wie 5-Chlorisatin, 5-Methoxyisatin, 5-Nitroisatin, 6-Nitroisatin, Isatin-5-sulfonsäure, Isatin-4-carbonsäure und Isatin-5-carbonsäure,
- N-substituierte Isatin-Derivate, wie N-Methylisatin, N-(2-Hydroxyalkyl)-isatin, N-(2-Hydroxypropyl)-isatin, N-(3-Hydroxypropyl)-isatin, N-(2,3-Dihydroxypropyl)-isatin, N-(2-Sulfoethyl)-isatin, (3-Sulfopropyl)-isatin, N-Allylisatin, N-Vinylisatin, N-Benzylisatin, N-(4-Methoxybenzyl)-isatin, N-(4-Carboxybenzyl)-isatin, N-(4-Sulfobenzyl)-isatin, N-(2-Dimethylaminoethyl)-isatin, N-(2-Pyrrolidinoethyl)-isatin, N-(2-Piperidinoethyl)-isatin, (2-Morpholinoethyl)-isatin, N-(2-Furylmethyl)-isatin, N-(Thien-2-ylmethyl)-isatin, N-(Pyrid-2-ylmethyl)-isatin, N-(Pyrid-3-ylmethyl)-isatin, N-(Pyrid-4-ylmethyl)-isatin, N-Allylisatin-5-sulfonsäure, 5-Chlor-N-(2-hydroxyethyl)-isatin, 5-Methyl-N-(2-hydroxyethyl)-isatin, 5,7-Dichlor-N-allylisatin, 5-Nitro-N-allylisatin, N-Hydroxymethylisatin, N-Hydroxymethyl-5-methylisatin, N-Hydroxymethyl-5-chlorisatin, N-Hydroxymethyl-5-sulfoisatin, N-Hydroxymethyl-5-carboxyisatin, N-Hydroxymethyl-5-nitroisatin, N-Hydroxymethyl-5-bromisatin, N-Hydroxymethyl-5-methoxyisatin, N-Hydroxymethyl-5,7-dichlorisatin, N-Dimethylaminomethylisatin, N-Diethylaminomethylisatin, N-(Bis-(2-hydroxyethyl)-aminomethyl)-isatin, N-(2-Hydroxyethylaminomethyl)-isatin, N-(Bis-(2-hydroxypropyl)-aminomethyl)-isatin, N-Pyrrolidinomethylisatin, N-Piperidinomethylisatin, N-Morpholinomethylisatin, N-(1,2,4-Triazolyl)-methylisatin, N-(1-Imidazolyl)-methylisatin, N-Carboxymethylaminomethylisatin, N-(2-Carboxyethylaminomethyl)-isatin, N-(3-Carboxypropylaminomethyl)-isatin, N-(Bis-(2-hydroxyethyl)-aminomethyl)-5-methylisatin, N-Piperidinomethyl-5-chlorisatin, N-(2-Sulfoethylamino)-isatin, sowie die Alkali- und gegebenenfalls Ammoniumsalze der sauren Verbindungen, Chinisatin und deren Derivate, wie N-Methylchinisatin,
- Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxy-acetophenon, 4-Amino-acetophenon, 4-Dimethylamino-acetophenon, 4-Morpholino-acetophenon, 4-Piperidino-acetophenon, 4-lmidazolino-acetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxy-benzophenon, 2-Amino-benzophenon, 4,4'-Dihydroxy-benzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, Indanon, 9-Fluorenon, 3-Hydroxyfluorenon, Anthron, 1,8-Dihydroxyanthron,
- heterocyclische Carbonylverbindungen, wie 2-lndolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1,3,3-Trimethyl-2-indolinyliden)-acetaldehyd, 1-Methylpyrrol-2-aldehyd, 1-Methyl-2-acetylpyrrol, 1-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5-Nitro-2-furyl)-acrolein, 3-(2-Furyl)-acrolein, Imidazol-2-aldehyd, Thiophen-2-aldehyd, 5-Methylthiophen-2-aldehyd, 5-Nitrothiophen-2-aldehyd, Carbazolaldehyde oder Carbazolketone, insbesondere 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd,
sowie den physiologisch verträglichen Salzen der vorgenannten Verbindungen.

13. Mittel nach einem der Ansprüche 1 bis 12, **dadurch gekennzeichnet, daß** die Komponente B ausgewählt ist aus der Gruppe bestehend aus N-(2-Hydroxyethyl)-N-ethyl-p-phenylendiamin, 2-Chlor-p-phenylendiamin, N,N-Bis-(2-hydroxyethyl)-pphenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-ptoluolsulfonat, 1-Methyl-2-chinaldinium-p-toluolsulfonat, 1,4-Dimethylchinolinium-p-toluolsulfonat, β-Alanin, L-Prolin, L-Lysin, DL-Tyrosin sowie deren vorzugsweise mit anorganischen Säuren gebildeten physiologisch verträglichen Salze, Vanillin, 5-Nitrovanillin, 4-Hydroxybenzaldehyd, 2,4-Dihydroxybenzaldehyd, 2,4-Dinitrobenzaldehyd, 4-Dimethylaminobenzaldehyd, 4-Pyrrolidino-benzaldehyd, 4-Diethylamino-2-hydroxy-benzaldehyd, 4-Trimethylammoniobenzaldehyd-methansulfonat, 4-Trimethylammoniobenzaldehyd-p-toluolsulfonat, 4-Trimethylammoniobenzaldehyd-methylsulfat, 4-Hydroxynaphthaldehyd, 4-Dimethylaminonaphthaldehyd, Salicylaldehyd, 4-Dimethylaminozimtaldehyd, Piperonal, 5-Nitropiperonal, 4-Nitrosalicylaldehyd, Coniferylaldehyd, 4-Formyl-1-methylpyridinium-benzolsulfonat, 4-Formyl-1-methylpyridinium-p-toluolsulfonat, 4-Formyl-1-methylpyridinium-iodid, 4-Benzoyl-1-methylpyridinium-methansulfonat, 4-Benzoyl-1-methylpyridinium-p-toluolsulfonat, 4-Formyl-1-methyl-chinoliniummethansulfonat, 4-Formyl-1-methyl-chinolinium-p-toluolsulfonat, 4-Formyl-1-methylchinolinium-trifluormethansulfonat, 2-Formyl-1-methyl-chinolinium-methansulfonat, 2-Formyl-1-methyl-chinolinium-p-toluolsulfonat, 2-Formyl-1-methyl-chinoliniumtrifluormethansulfonat, Chinolin-2-aldehyd, Chinolin-4-aldehyd, Indol-9-aldehyd, 1-Methyl-indol-3-aldehyd, 3-Azafluorenon, 3-Methyl-5-oxo-indano[2,1-b]pyridinium-ptoluolsulfonat, 3-Methyl-5-oxo-indano[2,1-b]pyridinium-trifluormethansulfonat, Antipyrin-4-aldehyd, Isatin, N-Vinylisatin, N-Allylisatin, Isatin-5-sulfonsäure (Natrium Salz), 5-(4-Dimethylaminophenyl)-penta-2,4-dienal, 9-Ethylcarbazol-3-carbaldehyd, 1,3-Diacetylbenzol, 1,3,5-Triacetylbenzol, 2-(2-Pyridoyl)-acetophenon, Salicil, 2,2'-Pyridil, 4-Hydroxyacetophenon, 2,4-Dihydroxyacetophenon, Furfural, 5-Nitrofurfural, 3-(5-Nitro-2-furyl)acrolein, Thiophen-2-aldehyd, 5-Nitrothiophen-2-aldehyd, 4-Pyridinaldehyd, 1-Methyl-4-[2-(4-formylphenyl)ethenyl]pyridinium-p-toluolsulfonat, 1-Methyl-4-[2-(4-formylphenyl)ethenyl]pyridinium-methylsulfat, 1-Methyl-2-[2-(4-formylphenyl)ethenyl]pyridinium-p-toluolsulfonat, 1-Methyl-2-[2-(4-formylphenyl)ethenyl]-pyridinium-methylsulfat, 2-(4-Formylphenyl)-3-methylbenzothiazolium-p-toluolsulfonat, 2-(4-Formylphenyl)-3-methylbenzothiazoliummethylsulfat, Pyridoxal und 2-(1,3,3,-Trimethyl-2-indolinyliden)acetaldehyd sowie den physiologisch verträglichen Salzen der vorgenannten Verbindungen.

14. Mittel zum Färben von keratinhaltigen Fasern, insbesondere menschlichen Haaren, enthaltend mindestens ein Reaktionsprodukt aus einer Komponente A und einer Komponente B als direktziehenden Farbstoff.

15. Mittel nach Anspruch 14, **dadurch gekennzeichnet, daß** das Reaktionsprodukt in einer Menge von 0,03 bis 65 mmol, insbesondere von 1 bis 40 mmol, bezogen auf 100 g des gesamten Färbemittels, enthalten ist.

16. Mittel nach einem der Ansprüche 1 bis 15, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

17. Mittel nach einem der Ansprüche 1 bis 16, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

18. Mittel nach einem der Ansprüche 1 bis 17, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

19. Mittel nach einem der Ansprüche 1 bis 18, **dadurch gekennzeichnet, daß** es Oxidationsmittel, insbesondere H₂O₂, in einer Menge von 0,01 bis 6 Gew.-%, bezogen auf die Anwendungslösung, enthält.

20. Mittel nach einem der Ansprüche 1 bis 19, **dadurch gekennzeichnet, daß** es anionische, zwitterionische oder nichtionische Tenside enthält.

21. Mittel nach einem der Ansprüche 1 bis 20, **dadurch gekennzeichnet, daß** es mindestens ein Oxidationsfarbstoffvorprodukt enthält.

22. Verwendung einer Kombination von Komponente A und Komponente B gemäß Anspruch 1 und/oder einem Reaktionsprodukt gemäß Anspruch 14, als eine färbende Komponente in Haarfärbemitteln.

23. Verfahren zum Färben von keratinhaltigen Fasern, insbesondere menschlichen Haaren, worin ein Färbemittel gemäß Anspruch 1, das zusätzlich übliche kosmetische Inhaltsstoffe enthält, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.
